# EUROPEAN PATENT SPECIFICATION

(11) **EP 4 447 938 B1**
(45) Date of publication and mention of the grant of the patent: **04.02.2026**
(21) Application number: 22836146.5
(22) Date of filing: 14.12.2022
(51) Int. Cl.: A61K 9/20, A24B 13/00, A24B 15/16, A61K 31/465

(54) **NOVEL HOLLOW ORAL NICOTINE PRODUCT**
NEUES HOHLES ORALES NIKOTINPRODUKT
NOUVEAU PRODUIT ORAL CREUX À BASE DE NICOTINE

(30) Priority: 16.12.2021 EP 21215245
(43) Date of publication of application: 23.10.2024
(73) Proprietor: Philip Morris Products S.A., 2000 Neuchâtel (CH)
(72) Inventor: CALI, Ricardo, 68163 Mannheim (DE); CHEUNG, Yiu Chi, Hong Kong (CN)
(74) Representative: Reddie & Grose LLP
(86) International application number: PCT/EP2022/085795
(87) International publication number: WO 2023/110984

(56) References cited:
- WO-A1-2004/064811
- WO-A2-2013/109931

## Description

The present invention relates to an oral nicotine product and to a method of producing such an oral nicotine product.

Smokeless oral tobacco products, such as snus and snuff, are well known in the art and have for many years provided an oral alternative to traditional smoking products. More recently, a new class of 'modern' oral nicotine products has emerged, which provide a source of nicotine that is typically free from tobacco. Oral nicotine products provide an alternative source of nicotine to e-cigarettes and aerosol-generating devices that deliver aerosolised nicotine through the heating of a nicotine source. One of the most common types of oral nicotine product is the tobacco-free nicotine pouch, which provides a nicotine powder composition inside a small, sealed pouch. During use, nicotine pouches are placed between the lip and the gum of the consumer, so that nicotine can be delivered into the body through the oral mucosal tissues. EP-A-1 617 823 discloses an oral pouch product containing a composition material comprising a biologically active agent such as nicotine, which is ionically bonded to an ionic carbohydrate. The pouch is adapted to provide rapid transmucosal delivery of the nicotine in the consumer's mouth.

Oral nicotine products in the form of lozenges, chewable tablets and chewing gum are also known. For example, WO-A-2017/182084 discloses an oral dosage form comprising nicotine-loaded cation exchange resin particles, wherein the oral dosage form may be a chewing gum, a tablet or a lozenge.

In general, oral nicotine products are not adapted to be swallowed but are retained in the mouth for a certain duration of time whilst the nicotine is released. After use, the consumer must therefore remove the residual product from their mouth and dispose of it. This handling of the oral nicotine product after use may be considered unappealing or inconvenient to some consumers, in particular, where immediate disposal of the used product is not possible. There may additionally be perceived issues with inconvenience or lack of hygiene in the handling of the oral nicotine products prior to use, during the removal of the products from the packaging and the insertion into the consumer's mouth.

It would therefore be desirable to provide an alternative form of oral nicotine product that can be consumed in a different way to existing products, providing a novel consumer experience. For example, it would be particularly desirable to provide such an oral nicotine product that can be used with reduced contact with the consumer's fingers, thereby providing potential improvements in hygiene and convenience. It would be further desirable to provide such a product that offers some flexibility to the consumer in terms of how it can be consumed. It would also be desirable to provide such a product that can be efficiently produced in a large scale manufacturing process.

WO 2013/109931 describes an oral product comprising a body comprising a mouth-soluble polymer matrix, cellulosic fibres embedded in the matrix, and nicotine or a derivative thereof dispersed in the matrix. The oral product is configured to release nicotine or a derivative thereof when the body is received within the oral cavity and exposed to saliva.

The present invention relates to an oral nicotine product formed of a nicotine composition.

According to the invention there is provided an oral nicotine product formed of a nicotine composition comprising a water soluble fibre matrix, a gum binder and a source of nicotine dispersed within the water soluble fibre matrix, wherein the oral nicotine product is in the form of a hollow tube defining a central longitudinal channel extending between the ends of the hollow tube and having a diameter of at least 4 millimetres, wherein the inner surface of the hollow tube comprises a plurality of longitudinal grooves extending between the ends of the hollow tube and wherein the longitudinal grooves have a depth of at least 1 millimetre.

The present invention also relates to a method for producing an oral nicotine product.

According to the invention there is provided a method for producing an oral nicotine product according to any preceding claim, the method comprising the steps of: forming a mixture comprising soluble fibre, a source of nicotine and a gum binder; heating the mixture to a temperature of less than 100 degrees Celsius; pouring the mixture into a tubular mould; cooling the mixture within the tubular mould in order to solidify the mixture in the form of a hollow tube; and removing the solidified hollow tube from the mould.

The present invention therefore provides an oral nicotine product having a novel, hollow tubular form. This hollow tubular form enables the oral nicotine product to be mounted and retained on some type of 'stick' whilst it is consumed, instead of inserting the product directly into the consumer's mouth. For example, the oral nicotine products according to the invention could be conveniently mounted on the end of a common object such as a straw, pencil, pen or chopstick. The consumer is then able to suck, chew or lick the oral nicotine product in order to achieve delivery of nicotine into their mouth.

This novel way of consuming the oral nicotine product of the present invention advantageously reduces contact between the oral nicotine product and the consumer's fingers. This in turn minimises risk of contamination of either the product itself, or the consumer's hands. Once in place on the selected 'stick', the product can be consumer discretely and conveniently and the consumer is provided with total control over the timing and duration of consumption. In this way, the consumer has greater flexibility over the use of the product.

As described below, certain embodiments of the invention provide oral nicotine products that can be held on the tip of the consumer's finger. This enables a certain level of transdermal delivery of the nicotine, in addition to the nicotine that is delivered orally, which optimises the overall delivery of nicotine to the consumer. Such embodiments also provide increased flexibility to the consumer in terms of how the nicotine is delivered.

The oral nicotine products according to the present invention can readily be manufactured using known moulding techniques.

As defined above, the oral nicotine products according to the present invention are in the form of a hollow tube. Preferably, the hollow tube has a cylindrical shape. Preferably, the outer transverse cross section of the hollow tube is substantially circular.

Preferably, the external diameter of the hollow tube is at least about 7 millimetres, more preferably at least about 8 millimetres and more preferably at least about 10 millimetres.

Preferably, the external diameter of the hollow tube is less than about 25 millimetres, more preferably less than about 15 millimetres and more preferably less than about 12 millimetres. For example, the hollow tube may have an external diameter of between about 7 millimetres and about 25 millimetres, or between about 8 millimetres and about 15 millimetres, or between about 10 millimetres and about 12 millimetres.

The hollow tube provides a central longitudinal channel defining a hole that passes all of the way through the hollow tube, between the two ends and which therefore enables the oral nicotine products to be inserted onto a stick like object, such as a straw or finger. The central longitudinal channel has a diameter of at least about 4 millimetres. The diameter is measured transversely to the longitudinal direction of the channel and corresponds to the 'internal diameter' of the hollow tube. Where the cross-sectional shape of the longitudinal channel is non circular so that the diameter of the longitudinal channel varies depending upon the angle of measurement, the minimum diameter is at least about 4 millimetres. This means that wherever the diameter is measured, it will always be at least about 4 millimetres. The provision of a longitudinal channel that has a diameter of 4 millimetres or greater enables the oral nicotine product to be effectively retained on a wider variety of common stick like objects, such as straws, pencils, etc.

Preferably, the diameter of the longitudinal channel of the hollow tube is at least about 5 millimetres, more preferably at least about 6 millimetres.

Preferably, the diameter of the longitudinal channel of the hollow tube is less than about 20 millimetres, more preferably less than about 15 millimetres and more preferably less than about 10 millimetres. For example, the longitudinal channel may have a diameter of between about 4 millimetres and about 20 millimetres, or between about 5 millimetres and about 15 millimetres, or between about 6 millimetres and about 10 millimetres.

The longitudinal channel of the hollow tube may have any suitable cross-sectional shape. Preferably, the longitudinal channel has a substantially circular transverse cross-section. This facilitates the insertion of the oral nicotine product onto a 'round' stick like object such as a straw, pencil or finger. The longitudinal channel preferably has a substantially constant transverse cross-section along its full length.

In certain preferred embodiments, the longitudinal channel is specifically adapted to receive a drinking straw, so that the oral nicotine product can be retained on the end of a straw during use. In other preferred embodiments, the longitudinal channel is specifically adapted to receive the end of the consumer's finger, so that the oral nicotine product can be retained on that finger during use.

The hollow tube preferably has a wall thickness at least about 2 millimetres, more preferably at least about 3 millimetres.

The hollow tube preferably has a length of at least about 8 millimetres, more preferably at least about 10 millimetres and more preferably at least about 15 millimetres.

Preferably, the hollow tube has a length of less than about 30 millimetres, more preferably less than about 25 millimetres and more preferably less than about 20 millimetres. For example, the length of the hollow tube may be between about 8 millimetres and about 30 millimetres, or between about 10 millimetres and about 25 millimetres, or between about 15 millimetres and about 20 millimetres.

Preferably, the hollow tube is elastically deformable up to applied forces of about 10 Newtons. This means that the hollow tube can be compressed with an applied force of up to 10 Newtons and will revert to substantially its original shape after the force is removed. This elastic property of the hollow tube allows the manipulation of the hollow tube in order to facilitate the mounting of the oral nicotine product on the corresponding 'stick'.

The nicotine composition forming the hollow tube, which will be described in more detail below, preferably has an elastic modulus of between about 1200 MPa and about 3800 MPa, more preferably between about 2100 MPa and about 3200 MPa.

In certain embodiments of the present invention, the hollow tube may be plastically deformable at applied forces of 12 Newtons and above. This means that when the hollow tube is compressed with an applied force of 12 Newtons or above, it will remain in its compressed form rather than reverting to the original form. This enables the oral nicotine product to be adapted to a flatter form that is more similar to the form of a conventional nicotine pouch. The compressed pouch can be conveniently inserted into the mouth in a similar way to a nicotine pouch, as an alternative to mounting the oral nicotine product on an elongate object. The consumer is therefore provided with increased flexibility in the how the oral nicotine product can be consumed.

In certain embodiments of the invention, the inner surface of the hollow tube, corresponding to the surface of the longitudinal channel, may be smooth or plain. In alternative embodiments of the invention, the inner surface of the hollow tube may be adapted to improve the grip between the hollow tube and the outer surface of the 'stick' on which the oral nicotine product is mounted. This may advantageously enable the oral nicotine product to be more effectively retained in place.

The inner surface of the hollow tube comprises a plurality of grooves or channels to provide improved grip. In particular, the inner surface of the hollow tube comprises a plurality of longitudinal grooves extending between the opposed ends of the hollow tube. Where a plurality of grooves are provided, the inner diameter of the hollow tube should be measured between the grooves, so that the inner diameter does not include the depth of the grooves.

The grooves have a depth of at least about 1 millimetre, more preferably at least about 1.5 millimetres. Preferably, the grooves have a depth of no more than about 3 millimetres, more preferably no more than about 2 millimetres. For example, the grooves may have a depth of between about 1 millimetres and about 3 millimetres, or between about 1 millimetre and about 2 millimetres, or between about 1.5 millimetres and about 3 millimetres, or between about 1.5 millimetres and about 2 millimetres.

As defined above, the hollow tube of the oral nicotine products of the present invention is formed of a nicotine composition comprising a water soluble fibre matrix and a source of nicotine dispersed within the water soluble fibre matrix.

The fibre matrix may be provided by any suitable fibrous material that is able to provide a matrix structure for retaining the source of nicotine within the nicotine composition. The fibrous material therefore provides a structure having a network of internal pores or channels within which the source nicotine can be contained. The fibrous material is water soluble so that it will gradually dissolve when the oral product is in contact with saliva inside the consumer's mouth. The fibre matrix therefore needs to be water soluble under the conditions typically provided within the consumer's mouth.

Preferably, the water soluble fibre matrix comprises cellulosic fibres. For example, the water soluble fibre matrix may comprise cellulosic fibres derived from wood pulp, cotton, sugar beets, bran, citrus pulp, switch grass, Salix, tea, populus, psyllium, arabinoxylan or any combination thereof. Alternatively, or in addition, the water soluble matrix may comprise one or more polysaccharides, for example, maltodextrin, inulin, starch, pectin, beta-glucans. Other suitable fibrous materials include oligosaccharides and lignin.

Preferably, the nicotine composition comprises at least about 20 percent by weight of the water soluble fibre matrix, more preferably at least about 30 percent by weight, on a dry weight basis.

Preferably, the nicotine composition comprises no more than about 60 percent by weight of the water soluble fibre matrix, more preferably no more than about 50 percent by weight, on a dry weight basis. For example, the nicotine composition may comprise between about 20 percent by weight and about 60 percent by weight of the water soluble fibre matrix, or between about 30 percent by weight and about 50 percent by weight, on a dry weight basis.

The source of nicotine contained within the water soluble fibre matrix may take any suitable form. Preferably, the source of nicotine comprises one or more nicotine salts. For example, the source of nicotine may comprise one or more nicotine salts selected from the list consisting of nicotine lactate, nicotine citrate, nicotine pyruvate, nicotine monotartrate, nicotine bitartrate, nicotine bitartrate dihydrate, nicotine benzoate, nicotine pectate, nicotine alginate, nicotine sulphate, nicotine hydrochloride and nicotine salicylate. Preferably, the source of nicotine comprises nicotine lactate or nicotine benzoate.

Alternatively, the source of nicotine may comprise liquid nicotine, nicotine gel, stabilised nicotine or nicotine base.

Preferably, the nicotine composition comprises at least about 0.5 percent by weight of the source of nicotine, more preferably at least about 1 percent by weight, more preferably at least about 1.5 percent by weight, on a dry weight basis.

Preferably, the nicotine composition comprises no more than about 10 percent by weight of the source of nicotine, more preferably no more than about 8 percent by weight, more preferably no more than about 6 percent by weight, on a dry weight basis. For example, the nicotine composition may comprise between about 0.5 percent by weight and about 10 percent by weight of the source of nicotine, or between about 1 percent by weight and about 8 percent by weight, or between about 1.5 percent by weight and about 6 percent by weight, on a dry weight basis.

Preferably, each individual oral nicotine product contains between about 1 mg and about 11 mg of nicotine. The amount of nicotine in the product can be varied depending upon the desired strength. For example, a 'light' strength product may contain between about 1 mg and about 3 mg of nicotine, a 'normal' strength product may contain between about 3 mg and about 7 mg of nicotine and a 'strong' product may contain between about 7 mg and about 11 mg of nicotine.

As defined above, the nicotine composition forming the hollow tube further comprises a gum binder. The gum binder is preferably water soluble. The inclusion of a gum binder in the oral nicotine product of the present invention may advantageously provide a desired level of elasticity and texture, which enables the product to be manipulated so that it can be inserted on the desired 'stick' by the consumer.

Suitable gum binders would be known to the skilled person and include but are not limited to natural gums such as gum Arabic, xanthan gum, gellan gum and combinations thereof.

The nicotine composition preferably comprises at least about 10 percent by weight of the gum binder, more preferably at least about 15 percent by weight, on a dry weight basis.

Preferably, the nicotine composition comprises no more than about 30 percent by weight of the gum binder, more preferably no more than about 25 percent by weight, on a dry weight basis. For example, the nicotine composition may comprise between about 10 percent by weight and about 30 percent by weight of the gum binder, or between about 15 percent by weight and about 25 percent by weight, on a dry weight basis.

Optionally, the nicotine composition may further comprise one or more flavourants. For example, it may be advantageous to provide one or more volatile flavours within the nicotine composition that are released to the consumer as the temperature of the oral nicotine product increases during use. This may enhance the sensorial experience of the consumer. The one or more flavourants may be provided in liquid form, or in gel form. The flavourants may be natural flavourants of synthetic flavourants. The one or more flavourants may be incorporated within the water soluble fibre matrix, together with the source of nicotine, or they may be applied to the nicotine containing fibre matrix.

Suitable flavourants would be known to the skilled person and include but are not limited to liquorice, wintergreen, cherry and berry type flavorants, Drambuie, bourbon, scotch, whiskey, spearmint, peppermint, lavender, cinnamon, cardamon, apium graveolents, clove, cascarilla, nutmeg, sandalwood, bergamot, geranium, honey essence, rose oil, vanilla, lemon oil, orange oil, Japanese mint, cassia, caraway, cognac, jasmin, chamomile, menthol, ylang ylang, sage, fennel, pimenta, ginger, anise, chai, coriander, coffee, mint oils from a species of the genus *Mentha,* cocoa, and combinations thereof.

Preferably, the nicotine composition comprises no more than about 4 percent by weight of flavourant or flavourants.

The level of flavourant can be adjusted below this level depending on the desired strength of flavour. Preferably, the nicotine composition comprises at least about 0.5 percent by weight of flavourant or flavourants.

Alternatively or in addition to the inclusion of one or more flavourants, the nicotine composition may optionally further comprise one or more sweeteners. The inclusion of one or more sweeteners may advantageously enhance the flavour of the oral nicotine product.

Suitable sweeteners would be known to the skilled person and include but are not limited to saccharine, sucralose, aspartame, acesulfame potassium, and combinations thereof.

Preferably, the nicotine composition comprises no more than about 2 percent by weight of the sweetener or sweeteners.

The level of sweetener can be adjusted below this level depending on the desired sweetness. Preferably, the nicotine composition comprises at least about 0.25 percent by weight of the sweetener or sweeteners.

Preferably, the nicotine composition further comprises a pH adjusting agent, which is a substance that adjusts the pH of the aqueous liquid, such as saliva, when the nicotine composition is dissolved in that aqueous liquid. Suitable pH adjusting agents include but are not limited to carbonates including monocarbonate, bicarbonate and sesquicarbonate, acetates, glycinates, gluconates, borates, glycerophosphates or citrates of alkaline metals or ammonium, phosphate systems including monohydrogenphosphate, dihydrogenphosphate and trihydrogenphosphate, metal hydroxides, such as sodium hydroxide and potassium hydroxide, and mixtures thereof. Preferred pH adjusting agents are sodium bicarbonate and sodium carbonate, and mixtures thereof.

The nicotine composition may include one or more optional additives. For example, the nicotine composition may include one or more of: non-nicotine alkaloids, minerals, vitamins, nutraceuticals, colourants, amino acids, antioxidants and botanicals.

In certain embodiments of the invention, the hollow tube may comprise a coating layer applied on at least one of its surfaces. Preferably, the coating layer is water soluble and therefore dissolvable in water, such that it readily dissolves upon contact with saliva when the oral nicotine product is inserted into the consumer's mouth during use.

Preferably, a coating layer is applied to at least the outer surface of the hollow tube. The coating layer provides a barrier layer which protects the nicotine composition and may help to prevent loss of active compounds, such as nicotine or flavourant, from the oral nicotine product prior to use.

Suitable coating materials for providing a coating layer on the hollow tube would be known to the skilled person and include but are not limited to carnauba wax, beeswax, gelatin, acetylated monoglyceride, starch (e.g., native potato starch, high amylose starch, hydroxypropylated potato starch), Zein, shellac, ethyl cellulose, methylcellulose, hydroxypropyl methylcellulose, carboxymethyl cellulose, and combinations thereof.

One or more additives may optionally be added to the coating material in order to modify the properties of the coating layer. For example, the coating layer may comprise one or more of the following additives in order to improve the barrier properties of the coating layer against moisture or oxygen: miglycol, titanium dioxide, kaoline, bentonite. Alternatively or in addition, the coating layer may comprise one or more sugar alcohols, for example, sorbitol, mannitol, xylitol, erythritol, isomalt, maltitol, lactitol, or combinations thereof. The one or more sugar alcohols may be included in order to enhance the flavour of the coating material, or to improve the texture of the coating material.

Alternatively or in addition, the coating layer may comprise one or more plasticisers. Suitable plasticisers include but are not limited to propylene glycol, glycerol, vegetable oil, triglyceride, or a combination thereof.

The intended use of the oral nicotine products according to the present invention will now be described. As discussed above, the oral nicotine products of the present invention are provided in hollow tubular form so that they can be readily mounted on a suitable 'stick' and retained on the 'stick' during use. The appropriate selection of a 'stick' will depend upon the dimensions of the oral nicotine product and in particular, the diameter of the longitudinal channel but suitable examples may include a straw, pencil, pen or chopstick. The 'stick' could alternatively be in the form of a finger of the consumer.

During use, a selected 'stick' of a suitable size is inserted by the consumer into the longitudinal channel of the hollow tube. The term 'stick' is used herein to refer to any suitable elongate object which is of appropriate proportions for the oral nicotine product to be mounted and retained on it. Preferably the outer diameter of the stick substantially corresponds to the inner diameter of the hollow tube so that the hollow tube can be retained in position during use by means of the friction between the inner surface of the hollow tube and the outer surface of the stick. Once mounted on the stick, the consumer can insert the oral nicotine product into their mouth for the desired duration. Any coating layer that is present will dissolve upon initial contact with the saliva in the consumer's mouth. The water soluble fibre matrix will then begin to dissolve and break down, thereby releasing the source of nicotine and any other components that may be present, such as flavourants, into the consumer's mouth.

The oral nicotine product may be entirely consumed during a single period of time, or it may be consumed over a plurality of separate periods of time. This provides the consumer with greater flexibility over the timing and duration of the consumption of the product.

If the oral nicotine product is mounted on the consumer's finger, some delivery of nicotine may additionally be provided transdermally once the water soluble matrix has begun to break down due to insertion of the oral nicotine product into the consumer's mouth. In this way, the nicotine may be delivered both orally and transdermally, through the skin.

The oral nicotine products according to the invention may be produced using any suitable methods and apparatus. Preferably, the oral nicotine products are formed using a moulding process in order to achieve the desired hollow tubular shape. An extrusion or die casting process may also be suitable for providing the tubular structure.

The present invention further provides a method for producing the oral nicotine products according to the invention, as defined above. In a first step of this method, a mixture comprising soluble fibres and a source of nicotine is formed. This mixture will typically be in the form of a solution comprising water. Any other optional components that are required for the oral nicotine product can be added to the mixture during this step. The resultant mixture is then heated to a temperature of less than 100 degrees Celsius. Preferably, the heating step is carried out at a temperature of between about 45 degrees and about 75 degrees Celsius, more preferably between about 50 degrees Celsius to about 60 degrees Celsius, wherein the temperature is selected in order to achieve the desired viscosity of the mixture.

The heated mixture is subsequently poured or injected into a tubular mould. Suitable moulding apparatus for this step would be known to the skilled person but includes starch mogul apparatus, which is commonly used for moulding of confectionery products. In such methods, a tray of corn starch is provided and a mould former is used to stamp the desired shape (in this case, tubular) into the corn starch bed. Once the products have been solidified, as described below, the starch can be removed and reused. A process of this type has been described in EP-A-2645885.

The mixture within the tubular mould is cooled in order to solidify the mixture in the form of the hollow tube. This may take up to 15 to 25 hours. During solidification, the soluble fibres will form a water soluble matrix around the source of nicotine.

Finally, the solidified hollow tube can be removed from the mould and packaged as desired.

Specific embodiments will be further described, by way of example only, with reference to the accompanying drawings in which:
Figure 1 shows a perspective view of an oral nicotine product not according to the present invention;
Figure 2 shows a perspective view of an oral nicotine product according to the present invention;
Figure 3 shows an end view of the oral nicotine product shown in Figure 2;
Figure 4 shows a schematic representation of the oral nicotine product of Figures 2 and 3 in place on a drinking straw; and
Figure 5 shows the oral nicotine product of Figures 1 and 2 in a compressed form.

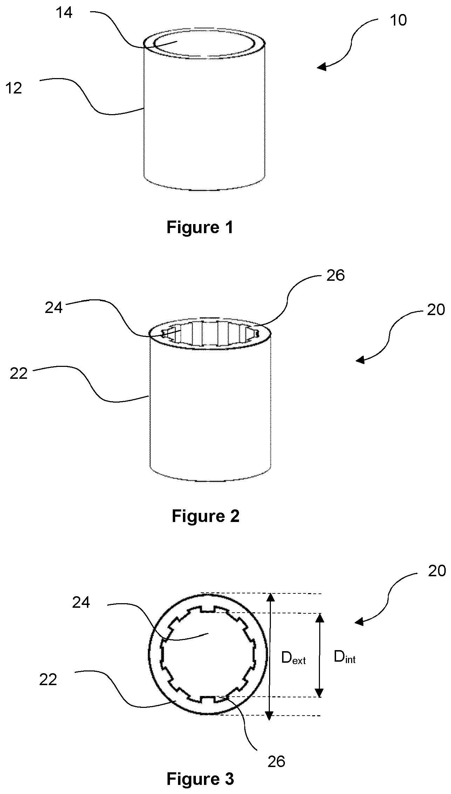

Figure 1 shows an oral nicotine product that is not according to the invention and has been described for background information only. The oral nicotine product shown in Figure 1 is in the form of a cylindrical hollow tube 10 which has a peripheral wall 12 formed of a nicotine composition and a central longitudinal channel 14 extending between the opposed ends of the hollow tube 10. The longitudinal channel 14 has a circular transverse cross section and the surface of the central longitudinal channel is plain. The diameter of the central longitudinal channel 14, which corresponds to the transverse internal diameter of the hollow tube 10 is 8 millimetres. The external diameter of the hollow tube 10 is 12 millimetres, such that the peripheral wall 12 has a thickness of 2 millimetres. The hollow tube 10 has a length of 18 millimetres. The hollow tube 10 is suitable for mounting on a stick like object such as a pen, chopstick or straw.

The hollow tube 10 is formed of a nicotine composition comprising a water soluble cellulose fibre matrix, nicotine salt, natural gum binder and flavourant. A dissolvable coating layer of beeswax (not shown) is provided on the outer surface of the hollow tube 10 in order to provide a barrier layer.

Figures 2 and 3 show an oral nicotine product according to the present invention, in the form of a hollow tube 20 comprising a peripheral wall 22 and a central longitudinal channel 24 between the opposed ends of the hollow tube 20. The hollow tube 20 is formed of a nicotine composition which is similar to that forming the hollow tube 10 shown in Figure 1, as described above. The longitudinal channel 24 has a circular transverse cross section but in contrast to the hollow tube 10, the inner surface of the longitudinal channel 24 comprises a plurality of longitudinal grooves 26 spaced apart around the circumference of the longitudinal channel 24 which provide a gripping surface. Each longitudinal groove 26 extends between the ends of the hollow tube 24 and has a substantially rectangular cross section, with a transverse depth of 1.5 millimetres. The external diameter (Dₑₓₜ) of the hollow tube 20 is 12 millimetres and the diameter of the longitudinal channel, corresponding to the internal diameter of the hollow tube (Dᵢₙₜ) is 9 millimetres. As shown in Figure 3, the internal diameter of the hollow tube (Dᵢₙₜ) is measured at a position away from the longitudinal grooves 26 and therefore does not take into account the depth of the longitudinal grooves 26. The value of Dᵢₙₜ is therefore the minimum internal diameter of the hollow tube. The hollow tube 20 has a length of 18 millimetres and is suitable for mounting on a stick like object such as a pen, chopstick or straw.

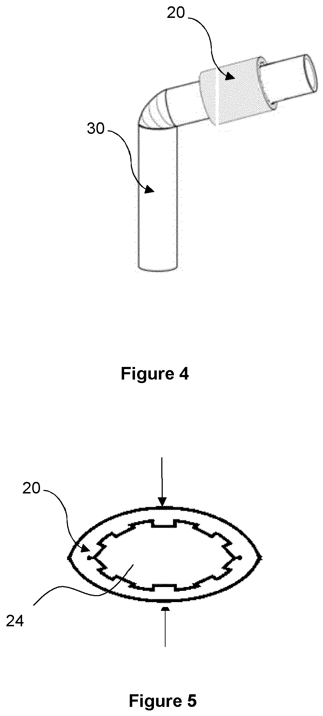

Figure 4 shows the hollow tube 20 of Figures 2 and 3 mounted on the end of a drinking straw 30, ready for consumption. The grooved inner surface of the hollow tube 20 grips the outer surface of the straw 30 so that the hollow tube can be retained in position on the straw during use, without slipping. During use, the consumer therefore does not need to touch the hollow tube 20 at all but can simply insert it into his mouth through movement of the straw 30.

The hollow tube 20 has a degree of elasticity so that it can be resiliently deformed to a certain degree, as a result of an applied compression by the consumer, in order to mount it on the desired 'stick' for consumption.

If desired, the hollow tube 20 can be compressed with a greater compressive force by the consumer to provide a permanently flatter form, as shown Figure 5, which more closely resembles the shape of an oral nicotine pouch. The compressive force should be applied by the consumer in the direction shown in Figure 5, at a level of above 12 Newtons. In this flattened form, the hollow tube 20 can be inserted into the mouth and retained between the gum and the lip in order to release the nicotine.

## Claims

1. An oral nicotine product formed of a nicotine composition comprising a water soluble fibre matrix, a gum binder and a source of nicotine dispersed within the water soluble fibre matrix, wherein the oral nicotine product is in the form of a hollow tube (20) defining a central longitudinal channel (24) extending between the ends of the hollow tube (20) and having a diameter of at least 4 millimetres,
wherein the inner surface of the hollow tube (20) comprises a plurality of longitudinal grooves (26) extending between the ends of the hollow tube (20) and wherein the longitudinal grooves (26) have a depth of at least 1 millimetre.

2. An oral nicotine product according to any preceding claim, wherein the nicotine composition has an elastic modulus of between 1200 and 3800 MPa.

3. An oral nicotine product according to any preceding claim, wherein the hollow tube (20) is elastically deformable under applied forces of up to 10 Newtons.

4. An oral nicotine product according to any preceding claim, wherein the hollow tube (20) has a wall thickness of at least 2 millimetres.

5. An oral nicotine product according to any preceding claim, wherein the central longitudinal cavity (24) has a diameter of between 6 millimetres and 10 millimetres.

6. An oral nicotine product according to any preceding claim, wherein the gum binder is water soluble.

7. An oral nicotine product according to any preceding claim, wherein the nicotine composition further comprises at least one flavourant.

8. An oral nicotine product according to any preceding claim, wherein the source of nicotine in the nicotine composition comprises one or more nicotine salts.

9. An oral nicotine product according to any preceding claim, wherein the water soluble fibre matrix comprises cellulosic fibres.

10. An oral nicotine product according to any preceding claim, further comprises a dissolvable coating layer applied on at least one surface of the hollow tube (20).

11. An oral nicotine product according to any preceding claim, wherein the hollow tube (20) is adapted to receive a drinking straw (30) in the longitudinal channel thereof.

12. An oral nicotine product according to any preceding claim, wherein the hollow tube (20) is adapted to be retained on the end of a consumer's finger.

13. A method for producing an oral nicotine product according to any preceding claim, the method comprising the steps of:
forming a mixture comprising soluble fibre, a source of nicotine and a gum binder;
heating the mixture to a temperature of less than 100 degrees Celsius;
pouring the mixture into a tubular mould;
cooling the mixture within the tubular mould in order to solidify the mixture in the form of a hollow tube (20); and
removing the solidified hollow tube (20) from the mould.

## Patentansprüche

1. Orales Nikotinprodukt, gebildet aus einer Nikotinzusammensetzung, umfassend eine wasserlösliche Fasermatrix, ein Gummibindemittel und eine Nikotinquelle, die innerhalb der wasserlöslichen Fasermatrix dispergiert ist, wobei das orale Nikotinprodukt in Form eines hohlen Rohres (20) vorliegt, das einen zentralen Längskanal (24) definiert, der sich zwischen den Enden des hohlen Rohres (20) erstreckt und einen Durchmesser von wenigstens 4 Millimeter aufweist,
wobei die Innenfläche des hohlen Rohrs (20) eine Vielzahl von Längsnuten (26) aufweist, die sich zwischen den Enden des hohlen Rohrs (20) erstrecken, und wobei die Längsnuten (26) eine Tiefe von wenigstens 1 Millimeter aufweisen.

2. Orales Nikotinprodukt nach einem beliebigen vorhergehenden Anspruch, wobei die Nikotinzusammensetzung einen Elastizitätsmodul zwischen 1200 und 3800 MPa aufweist.

3. Orales Nikotinprodukt nach einem beliebigen vorhergehenden Anspruch, wobei das hohle Rohr (20) unter aufgebrachten Kräften von bis zu 10 Newton elastisch verformbar ist.

4. Orales Nikotinprodukt nach einem beliebigen vorhergehenden Anspruch, wobei das hohle Rohr (20) eine Wandstärke von wenigstens 2 Millimeter aufweist.

5. Orales Nikotinprodukt nach einem beliebigen vorhergehenden Anspruch, wobei der zentrale Längshohlraum (24) einen Durchmesser zwischen 6 Millimeter und 10 Millimeter aufweist.

6. Orales Nikotinprodukt nach einem beliebigen vorhergehenden Anspruch, wobei das Gummibindemittel wasserlöslich ist.

7. Orales Nikotinprodukt nach einem beliebigen vorhergehenden Anspruch, wobei die Nikotinzusammensetzung ferner wenigstens einen Geschmacksstoff umfasst.

8. Orales Nikotinprodukt nach einem beliebigen vorhergehenden Anspruch, wobei die Nikotinquelle in der Nikotinzusammensetzung ein oder mehrere Nikotinsalze umfasst.

9. Orales Nikotinprodukt nach einem beliebigen vorhergehenden Anspruch, wobei die wasserlösliche Fasermatrix Zellulosefasern umfasst.

10. Orales Nikotinprodukt nach einem beliebigen vorhergehenden Anspruch, ferner aufweisend eine auflösbare Überzugsschicht, die auf wenigstens einer Fläche des hohlen Rohres (20) aufgebracht ist.

11. Orales Nikotinprodukt nach einem beliebigen vorhergehenden Anspruch, wobei das hohle Rohr (20) angepasst ist, um einen Trinkhalm (30) in seinem Längskanal aufzunehmen.

12. Orales Nikotinprodukt nach einem beliebigen vorhergehenden Anspruch, wobei das hohle Rohr (20) angepasst ist, um an dem Ende des Fingers eines Verbrauchers gehalten zu werden.

13. Verfahren für ein Herstellen eines oralen Nikotinprodukts nach einem beliebigen vorhergehenden Anspruch, das Verfahren umfassend die folgenden Schritte:
Bilden eines Gemisches, aufweisend lösliche Fasern, eine Nikotinquelle und ein Gummibindemittel;
Erwärmen des Gemisches auf eine Temperatur von weniger als 100 Grad Celsius;
Gießen des Gemischs in eine rohrförmige Form;
Abkühlen des Gemischs innerhalb der rohrförmigen Form, um das Gemisch in Form eines hohlen Rohrs (20) zu verfestigen; und
Entfernen des verfestigten hohlen Rohrs (20) aus der Form.

## Revendications

1. Produit buccal à base de nicotine formé d'une composition de nicotine comprenant une matrice de fibres soluble dans l'eau, un liant de gomme et une source de nicotine dispersée au sein de la matrice de fibres soluble dans l'eau, dans lequel le produit buccal à base de nicotine se présente sous la forme d'un tube creux (20) définissant un canal longitudinal (24) central s'étendant entre les extrémités du tube creux (20) et ayant un diamètre d'au moins 4 millimètres,
dans lequel la surface intérieure du tube creux (20) comprend une pluralité de rainures longitudinales (26) s'étendant entre les extrémités du tube creux (20) et dans lequel les rainures longitudinales (26) ont une profondeur d'au moins 1 millimètre.

2. Produit buccal à base de nicotine selon la revendication précédente, dans lequel la composition de nicotine a un module d'élasticité compris entre 1200 et 3800 MPa.

3. Produit buccal à base de nicotine selon l'une quelconque des revendications précédentes, dans lequel le tube creux (20) est élastiquement déformable sous des forces appliquées allant jusqu'à 10 Newtons.

4. Produit buccal à base de nicotine selon l'une quelconque des revendications précédentes, dans lequel le tube creux (20) a une épaisseur d'au moins 2 millimètres.

5. Produit buccal à base de nicotine selon l'une quelconque des revendications précédentes, dans lequel la cavité longitudinale centrale (24) a un diamètre compris entre 6 millimètres et 10 millimètres.

6. Produit buccal à base de nicotine selon l'une quelconque des revendications précédentes, dans lequel le liant de gomme est soluble dans l'eau.

7. Produit buccal à base de nicotine selon l'une quelconque des revendications précédentes, dans lequel la composition de nicotine comprend en outre au moins un aromatisant.

8. Produit buccal à base de nicotine selon l'une quelconque des revendications précédentes, dans lequel la source de nicotine dans la composition de nicotine comprend un ou plusieurs sels de nicotine.

9. Produit buccal à base de nicotine selon l'une quelconque des revendications précédentes, dans lequel la matrice de fibres soluble dans l'eau comprend des fibres cellulosiques.

10. Produit buccal à base de nicotine selon l'une quelconque des revendications précédentes, comprenant en outre une couche de revêtement soluble appliquée sur au moins une surface du tube creux (20).

11. Produit buccal à base de nicotine selon l'une quelconque des revendications précédentes, dans lequel le tube creux (20) est adapté pour recevoir une paille (30) dans son canal longitudinal.

12. Produit buccal à base de nicotine selon l'une quelconque des revendications précédentes, dans lequel le tube creux (20) est adapté pour être retenu sur l'extrémité du doigt d'un consommateur.

13. Procédé de production d'un produit buccal à base de nicotine selon l'une quelconque revendication précédente, le procédé comprenant les étapes consistant à :
former un mélange comprenant une fibre soluble, une source de nicotine et un liant de gomme ;
chauffer le mélange jusqu'à une température inférieure à 100 degrés Celsius ;
verser le mélange dans un moule tubulaire ;
refroidir le mélange au sein du moule tubulaire afin de solidifier le mélange sous la forme d'un tube creux (20) ; et
retirer le tube creux (20) solidifié du moule.
